(19) **Europäisches Patentamt**

**European Patent Office**

**Office européen des brevets**

(11) **EP 1 284 672 B1**

(12) **EUROPEAN PATENT SPECIFICATION**

| | |
|---|---|
| (45) Date of publication and mention of the grant of the patent:<br>**27.12.2006  Bulletin 2006/52** | (51) Int Cl.:<br>***A61B 18/18*** *(2006.01)*    ***A61B 18/22*** *(2006.01)* |
| (21) Application number: **00953703.6** | (86) International application number:<br>**PCT/US2000/020494** |
| (22) Date of filing: **27.07.2000** | (87) International publication number:<br>**WO 2001/008579 (08.02.2001 Gazette 2001/06)** |

(54) **DUAL WAVELENGTH MEDICAL DIODE LASER SYSTEM**

MEDIZINISCHES DIODENLASERSYSTEM MIT ZWEI WELLENLÄNGEN

SYSTEME LASER MEDICAL A DIODE A DOUBLE LONGUEUR D'ONDE

| | |
|---|---|
| (84) Designated Contracting States:<br>**AT BE CH CY DE DK ES FI FR GB GR IE IT LI LU MC NL PT SE** | (73) Proprietor: **CeramOptec GmbH**<br>**53121 Bonn (DE)** |
| (30) Priority:  **30.07.1999  US 364961** | (72) Inventor: **NEUBERGER, Wolfgang**<br>**87000 FT. Lubuan (MY)** |
| (43) Date of publication of application:<br>**26.02.2003  Bulletin 2003/09** | (56) References cited:<br>**FR-A- 2 584 199**    **US-A- 5 370 643**<br>**US-A- 5 451 221**    **US-A- 5 634 922**<br>**US-A- 5 668 903**    **US-A- 5 868 734** |

**Description**

**BACKGROUND OF THE INVENTION**

The present invention relates to diode laser systems that transmit at least two wavelengths or power densities of light energy through a single step index fiber system having at least two core sections.

The present use of lasers in medical procedures and surgical applications is virtually unlimited. Solid state lasers, such as the Nd:YAG, have primarily been employed to achieve the desired optical power and wavelength for medical procedures in the fields of dermatology, plastic surgery, ophthalmology, otolaryngology, neurological surgery, gastroenterology, urology, gynecology, and general surgery. Solid state lasers employed for these applications are expensive, complex and generally inefficient. Diode lasers are attractive substitutes for these solid state lasers because diode lasers are cheap, small, and have low power requirements. Individual diode lasers deliver relatively low optical power however, compared to solid state laser. To obtain effective power, these lasers are typically combined to form an array on one or more substrates.

Light energy focused on tissue during medical procedures results in local hyperthermia, coagulation, or vaporization depending on the power density of the radiation energy. A lower power density of light energy is required for local hyperthermia, which may be mediated through the activation of collagenase and may subsequently result in the destruction of local blood vessels. A higher power density of light energy is necessary for coagulation, which results from the denaturation of proteins. An even higher power density of light energy is essential for vaporization, which is the process of removing solid tissue by converting it into a gaseous vapor or plume. This process is also referred to as tissue ablation or cutting.

Ablating tissue can produce successive circumambient zones of carbonization, vacuolization and edema as the heat is dissipated. A small spot size of radiation minimizes edema, and causes less collateral damage to healthy cells surrounding the spot. A larger spot size is less precise and tends to coagulate the tissue. Thus, depending on the desired effect of a medical laser, a small or large spot size is chosen. The collective output power of a diode laser array or arrays must be concentrated onto a small area to be effective for medical applications such as cutting or vaporizing tissue. Highly concentrated light energy corresponds to a high power density, which may not however, be advantageous for all medical laser applications. In fact, for applications such as coagulation, high power density radiation is unfavorable. A single diode laser that is effectively transmitted is an advantage over a diode laser array.

The spot size is inversely proportional to the cross sectional core area of the optical fiber transmitting the light energy; i.e. a smaller cross section increases power density for a given power. Coupling large amounts of light energy into increasingly smaller fiber core cross sections can be difficult however, because only light entering the fiber core at an angle of incidence less than the critical angle will be refracted into the core. This phenomenon can be explained through Snell's Law:

$$n_1 \sin\Theta_1 = n_2 \sin\Theta_2 \qquad\qquad (1)$$

where $n_1$ is the refractive index of the medium, $\Theta_1$ is the angle of incidence defined relative to the normal, $n_2$ is the refractive index of the fiber core material, and $\Theta_2$ is the angle of refraction defined relative to the normal.

When light passes from a medium of larger refractive index into one of smaller refractive index - for example, from a fiber core to a fiber cladding - the refracted ray bends away from the normal of the core-cladding interface. Radiation propagating through the fiber core, that strikes the fiber cladding at an angle of incidence above a certain value (critical angle), is refracted perpendicular to the normal of the core-cladding interface. Therefore, all incident radiation is advantageously reflected back into the optical fiber core.

This elementary physical principle of total reflection has been exploited by Richard Nagal (Int. Pat. No. WO 95/15508) to construct a step index optical fiber section for transmission of a single wavelength of light. Nagal's invention describes a coupling device that allows for increased transmission of single wavelength light. Light that falls outside the acceptance angle and diameter of the central fiber core, enters the first cladding which functions as an additional core. The first cladding functions as a normal cladding as well as a second core. This first cladding layer has a refractive index less than the core and is surrounded by a second cladding layer. In effect, there are two concentric cores and two concentric claddings. Nagal however does not contemplate using this fiber section as a delivery fiber. Furthermore, the fiber section is only used to couple a single type of laser radiation to a standard delivery fiber. Nagal does not allow for introduction of more than one wavelength into the fiber or transmission of both high and low power densities.

Document US5668903 discloses a laser system for medical applications comprising a multicore combiner adapted to combine a plurality of laser beams output by a plurality of diode lasers into a single beam of high brightness and high power density.

Document US 5451221 describes an endoscopic light delivery system adapted to deliver light of different wavelengths to a tissue in order to simultaneously cut and coagulate the tissue.

Means to simultaneously cut and ablate tissue with radiation from the same fiber have been suggested in the prior art. However, these systems incorporate solid state laser systems, which are bulky, complicated, and expensive. Additionally, prior art systems rely on beam splitting methods, which have a decrease in power density and beam quality. Therefore, there is a need for a system that takes advantage of inexpensive diode lasers and is capable of transmitting both the high power density radiation (or highly absorptive wavelength) used for cutting or ablating as well as the lower power density radiation used for coagulation of surrounding tissue.

## OBJECTS AND SUMMARY OF THE INVENTION

It is therefore an object of the present invention to provide a medical diode laser system that allows for efficient transmission of at least two power densities or at least two wavelengths.

Another object of the present invention is to provide a medical diode laser system that allows for simultaneous coagulation, and cutting or ablating of tissue.

Still another object of the present invention is to provide a multiple core fiber which allows simultaneous transmission of both high power density radiation or high absorption wavelength that may be used for tissue cutting or ablating, and low power density radiation that may be used for tissue coagulation and/or bio-stimulation.

Briefly stated, the present invention provides a medical diode laser system that allows for simultaneous transmission of at least two wavelengths and at least two power densities. Radiation of a suitable wavelength for tissue cutting or ablating is coupled into an inner fiber core to produce an output beam with sufficient power density for ablation. Radiation of a suitable wavelength for tissue coagulation is introduced into an outer fiber core to produce another output beam with a lower power density appropriate for coagulation. The outer fiber core that immediately surrounds the inner fiber core has a refractive index less than the inner fiber core and thereby functioning as cladding for the inner core. Cladding that has a refractive index that is less than the outer fiber core surrounds the outer fiber core. Alternatively, material functioning as an intermediary cladding separates the outer and inner fiber cores. In this embodiment, the inner core has a larger refractive index than the inner cladding which in turn has a lower refractive index than the outer core, which is surrounded by an outer cladding also having a refractive index lower than the outer fiber core. In operation, both of these embodiments allow for transmission of high and low power density radiation that can be used for efficient cutting or ablation, bio-stimulation and coagulation of various tissues.

The above and other objects, features and advantages of the present invention will become apparent from the following detailed description read in conjunction with the accompanying drawings.

## BRIEF DESCRIPTION OF THE DRAWINGS

FIG. 1 displays an embodiment of the present invention in cross sectional view, irradiating a tissue surface.

FIG. 2 illustrates a top view of a laser spot made by embodiments shown in FIG. 1 and 4.

FIG. 3 shows a cross section of an embodiment with an additional cladding layer.

FIG. 4 shows a cross sectional view of fiber 18 that is shown in FIG. 1.

FIG. 5-8 illustrate further cross sections of embodiments of the present invention.

FIG. 9-10 illustrate further embodiments of the present invention in cross sectional view, irradiating a tissue surface and utilizing a prism to direct radiation.

## DETAILED DESCRIPTION OF PREFERRED EMBODIMENTS

The present invention provides a device that allows for concurrent transmission of at least two wavelengths or at least two power densities which can for example simultaneously cut or vaporize and coagulate tissue. The present invention allows two different wavelengths and power densities to be transmitted through the same fiber so that it is it possible for example to cut and coagulate simultaneously with a single medical instrument. The present invention allows multiple laser sources to be transmitted through a single optical delivery fiber. This is an advantage over using multiple fibers, because the diameter of a device using the present invention can be made much smaller and much easier to use. Furthermore, the present invention is not limited to transmission of only two wavelengths or power densities. Additional concentric layers can be added to the fiber to transmit additional wavelengths.

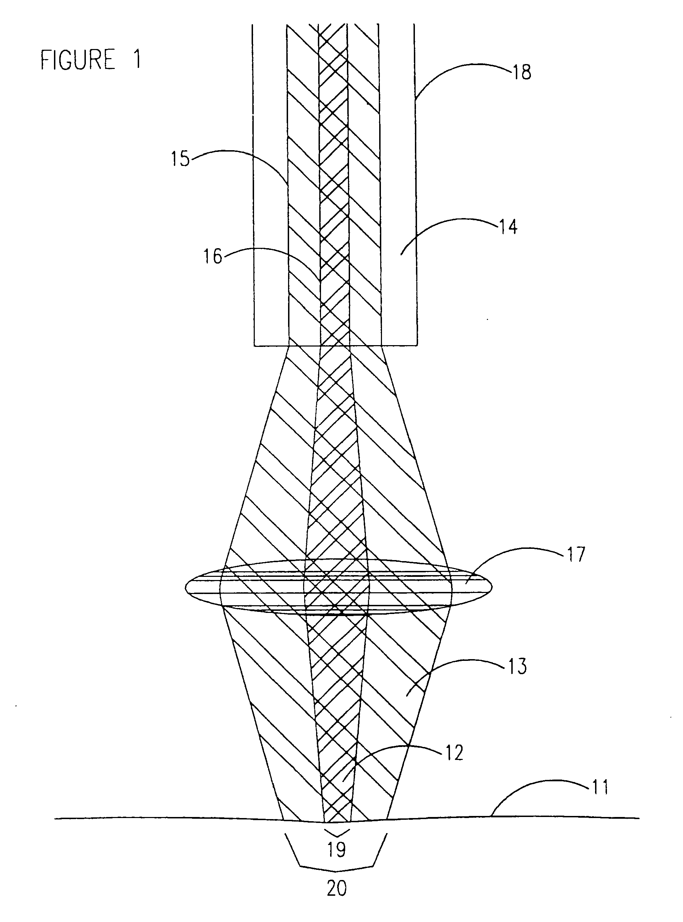

FIG. 1 illustrates an embodiment of this device, which can be used in many medical applications. Two different wavelengths of radiation are transmitted through fiber 18 and focused by lens 17 onto tissue 11. Inner core radiation 12, for example 1.9 $\mu$m wavelength radiation, is guided into and transmitted through inner fiber core 16 and focused by lens **17** to small spot **19.** Inner core radiation **12** has a wavelength chosen for tissue **11** absorption. Small spot **19** has sufficient power density to cut or ablate tissue **11.**

Outer core radiation **13** is used to coagulate tissue **11.** Outer core radiation **13** is guided into and transmitted through outer fiber core **15,** which encircles inner fiber core **16.** Outer fiber core **15** has a refractive index smaller than inner fiber core **16** and acts as a cladding to inner fiber core **16.** Similarly, cladding **14** has a smaller refractive index than outer fiber core **15.** Outer core radiation **13** is focused by lens **17** to large spot **20** on tissue **11.** Large spot **20** has a lower power density suitable for tissue **11** coagulation.

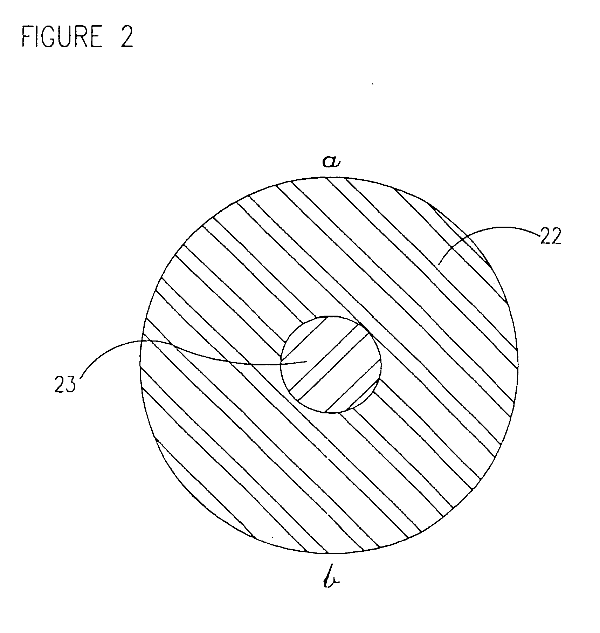

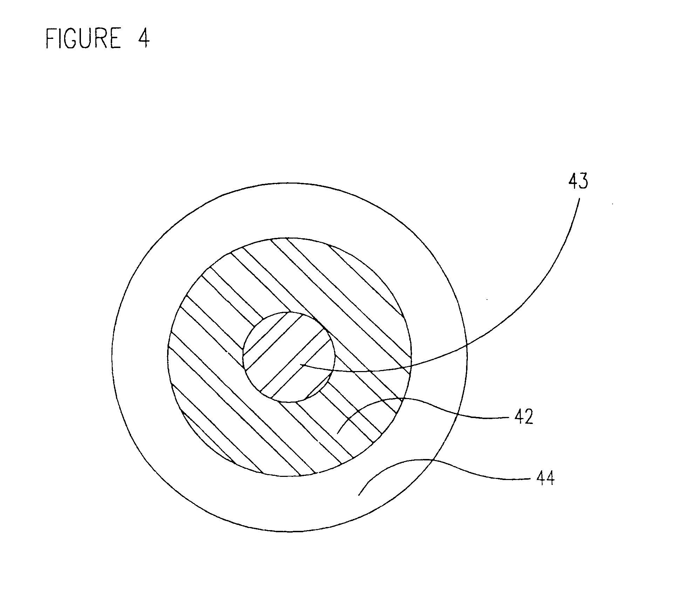

**FIG. 2** depicts a laser spot produced by radiation from embodiments depicted in **FIG. 1** and **4.** Radiation transmitted through the inner core is focused to small spot **23,** which corresponds to small spot **19** of **FIG. 1**. High power density radiation at small spot **23** is used to cut or ablate tissue. Large spot **22,** which corresponds to large spot **20** of **FIG. 1,** surrounds and encompasses small spot **23.** Radiation focused to large spot **22** coagulates tissue surrounding and encompassing the tissue that is cut or ablated by small spot **23.**

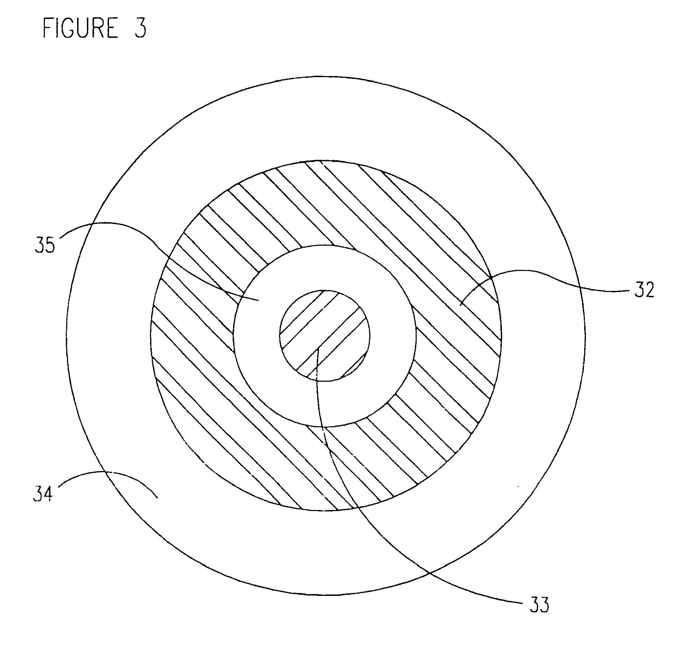

**FIG. 3** displays an embodiment of a side cut cross sectional view of an optical fiber. Normally optical fibers would have at least one buffer layer in addition to the structure in **FIG. 3,** to protect the optical fiber. In this embodiment four concentric layers constitute the part of the optical fiber that transmits two wavelengths of radiation. Inner fiber core **33** transmits high power density radiation and is surrounded by inner cladding **35.** Inner cladding **35** is surrounded by outer fiber core **32,** which transmits the second wavelength radiation. Outer cladding **34** forms the fourth layer and surrounds outer fiber core **32.** The refractive index of inner fiber core **33** is larger than the refractive index of inner cladding **35** which also has a smaller refractive index than outer fiber core **32.** Outer cladding **34,** which forms the fourth concentric layer has a refractive index smaller than outer fiber core **32.** In operation, the fiber cores are both surrounded by lower refractive index material, which creates total internal reflection inside the individual fiber cores. This total internal reflection has the added advantage of diminished cross-talk between cores.

**FIG. 4** shows another embodiment of a side cut cross sectional view of the fiber shown in **FIG. 1.** Inner fiber core **43,** which corresponds to **16** of **FIG. 1,** is surrounded by outer fiber core **42,** which corresponds, to **15** of **FIG. 1.** Cladding **44,** which corresponds to **14 of FIG. 1,** provides a guide for light propagating in outer fiber core **42.** This is because cladding **44** has a lower refractive index than outer fiber core **42.** Outer fiber core **42** in turn serves as a light guide for radiation propagating in inner fiber core **43** because outer fiber core has a lower refractive index than inner fiber core **43.**

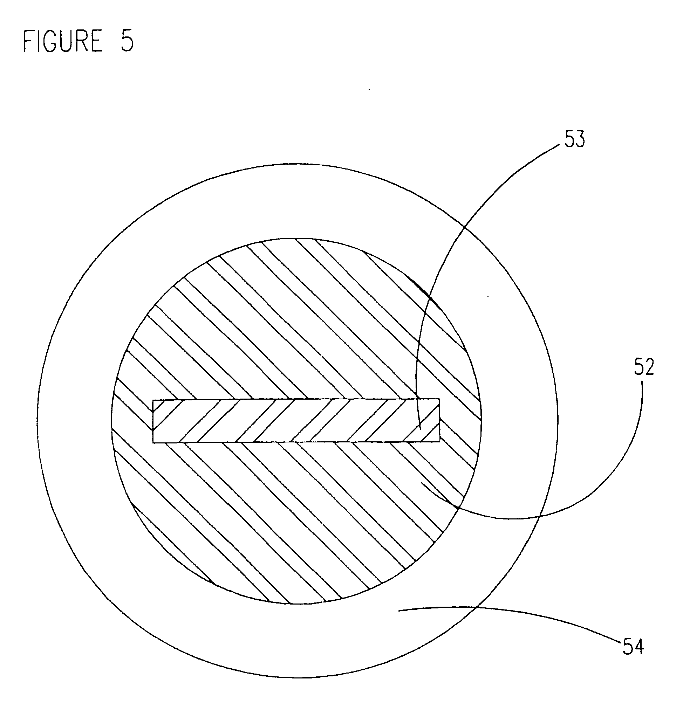

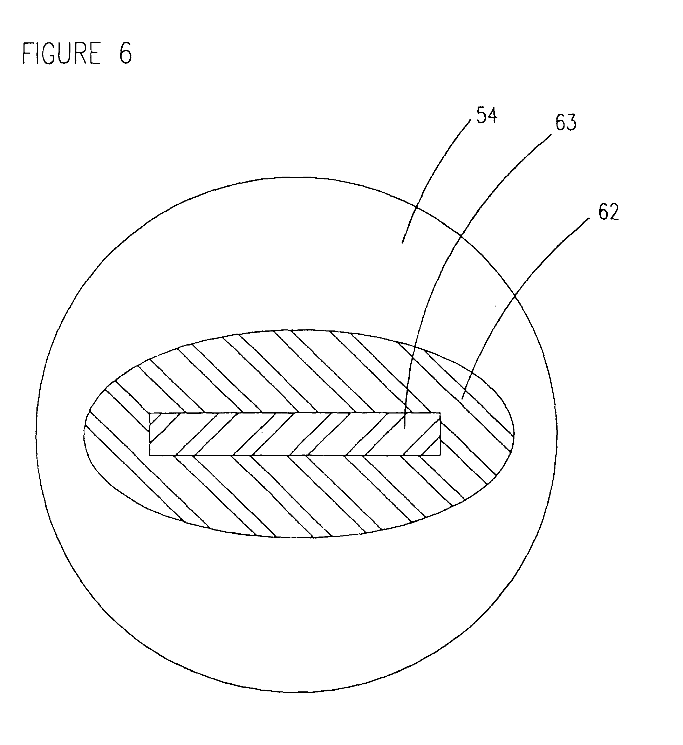

**FIG. 5** and **6** both show further embodiments in side cut cross sectional view which utilize different inner core **53** and **63** and outer core **52** and **62** shapes and sizes respectively. The cross sectional outer shape of cladding **54** and **64** remains constant although the cross sectional areas are different. **FIG. 4-6** all have an inner core with a refractive index greater than the outer core which in turn has a greater refractive index than the cladding layer.

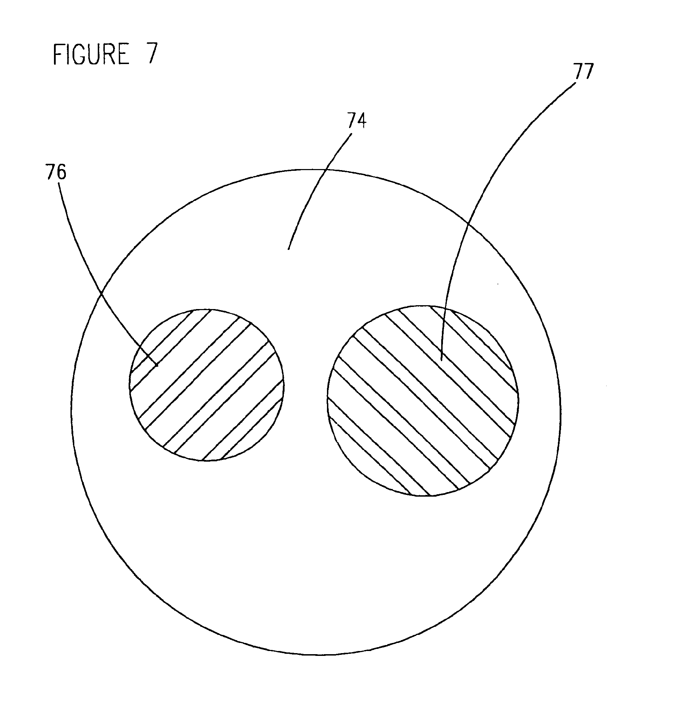

In **FIG. 7,** an embodiment is shown in a side cut cross sectional view where two separate cores with differing cross sectional areas lie within the cladding, allow two different wavelengths of light to propagate through a fiber. The cores are separated by cladding which keeps radiation from overlapping within the fiber. The cores depicted in **FIG. 7** have a circular cross sectional shape. In an alternative they are designed with an oval cross sectional shape. High power density radiation used for cutting or ablating is transmitted through small fiber core **76.** Large fiber core **77** transmits lower power density radiation used for coagulation. Small fiber core **76** has a smaller cross sectional area than large fiber core **77.** Cladding **74** surrounds both fiber cores.

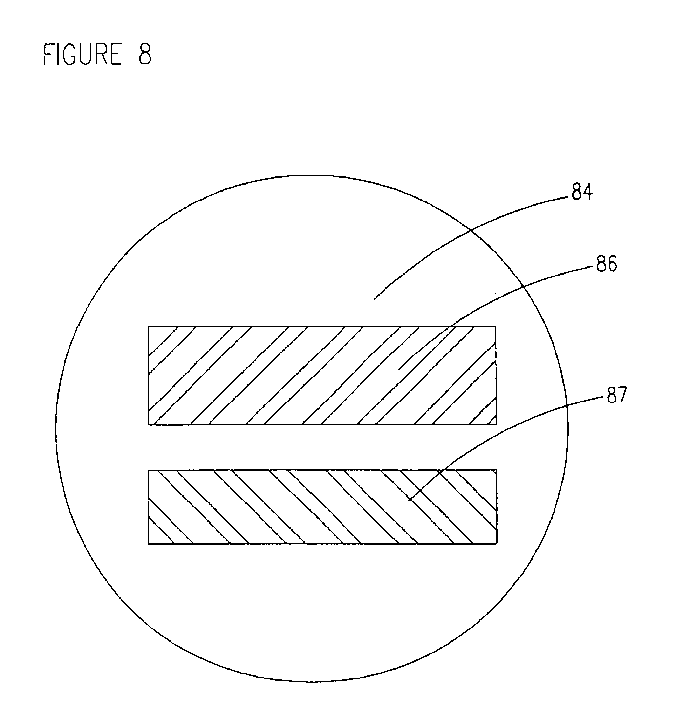

Similarly, **FIG. 8** depicts a side cut cross sectional view of another embodiment that utilizes rectangular fiber cores. A rectangular shape core cross section is an advantage when coupling a fiber to a diode laser. Since, the laser output from the diode laser has a distinctly rectangular shape, the rectangular core matches the output and provides a more efficient coupling. If the diode is more effectively coupled to the fiber, a lower power diode laser can be used to for applications. High power densities can be transmitted without significant loss at the coupling. Furthermore, relatively less power needs to be transmitted into the fiber for a particular output as compared to the input needed into a substantially circular core. Radiation used for example for cutting or ablating is transmitted through small fiber core **87,** which corresponds to **76** of **FIG. 7.** Radiation used for coagulating is transmitted through large fiber core **86,** which corresponds to **77** of **FIG. 7.** Cladding **84** surrounds both fiber cores.

In both **FIG. 7** and **8,** the fiber cores may have similar refractive indices, but the cladding must have a smaller refractive index. Embodiments shown in **FIG. 7** and **8** may be particularly useful in applications where a site must be cut or ablated, but an area immediately to one side of the site will be harmed by any radiation exposure. In this embodiment, the fiber may be turned so that coagulating radiation is directed to an area opposite the potentially harmed tissue site. In alternate embodiments either two different power densities or two different wavelengths are transmitted through the core sections. Two different densities of the same wavelength radiation are preferable for coagulation by one density and incision by the other. Two different wavelengths are preferable when one is used for incision or coagulation and the other wavelength is used for an application such as bio-stimulation.

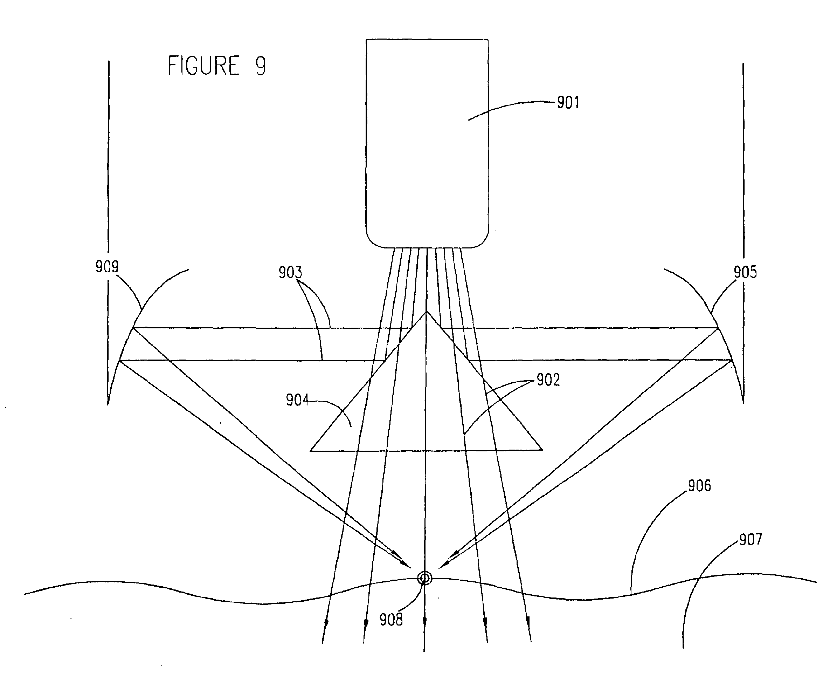

**FIG. 9** illustrates another embodiment of the present invention in cross sectional view, which simultaneously coagulates tissue and allows for a more precise incision site. Two different radiation wavelengths are transmitted through fiber **901** onto tissue **907.** Prism **904** has a suitable coating that discriminates between the two wavelengths that are transmitted through fiber **901.** Inner core radiation **903,** for example 1.9 μm wavelength radiation, is reflected off prism **904,** and directed to incision site **908** by reflecting optics **909** and **905.** Reflecting optics **909** and **905** are designed to focus inner core radiation **903** more precisely to create an incision. Outer core radiation **902** is used to coagulate tissue **907.** Outer core radiation **902** passes through prism **904** and irradiates tissue surface **908** and tissue **907.**

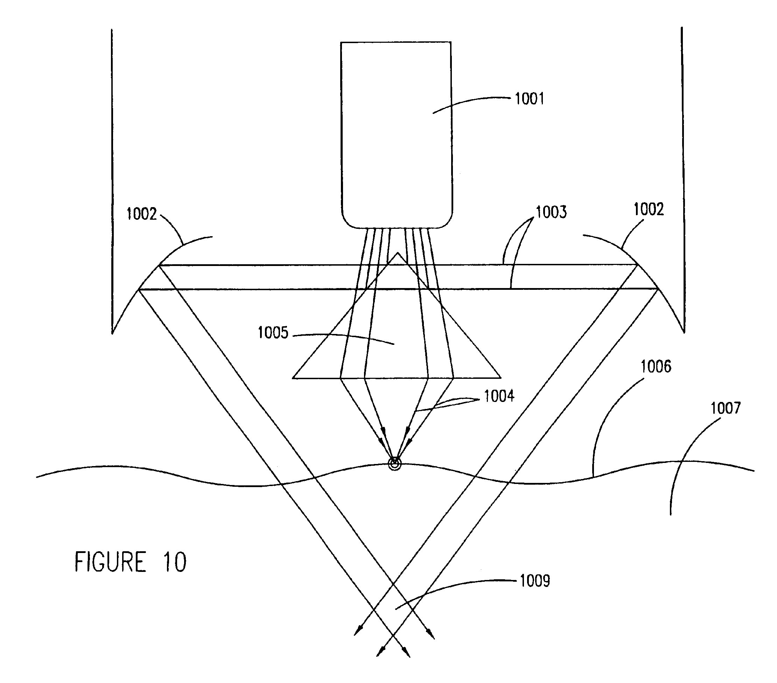

FIG. 10 depicts another embodiment that allows very precise sections of tissue to be either coagulated or incised. Two different wavelengths of radiation are transmitted through fiber **1001** onto tissue **1007** and tissue surface **1006.** Prism **1005** has a suitable coating that discriminates between the two wavelengths that are transmitted through fiber **1001.** Inner core radiation **1003** is reflected off prism **1005,** and directed to coagulation area **1009** by reflecting optics **1002.** Reflecting optics **1002** are designed to direct inner core radiation **1003** to an area within the tissue **1007** below tissue surface **1006.** Outer core radiation **1004** passes through prism **1005** and is directed to incision area **1008.**

In yet another embodiment of the present invention pulsed laser radiation is used for ablation, and continuous wave radiation is used for coagulation purposes. As stated earlier, power density plays an important role in determining the tissue effects. Radiation pulsed in intervals shorter than the thermal relaxation time of the tissue segment irradiated is typically used for ablative purposes, while coagulation is generally achieved by continuous wave radiation. In this alternative, the inner core area (in the concentric core arrangement described above) is used to transmit the pulsed radiation, while the surrounding outer core carries the continuous wave radiation.

Having described preferred embodiments of the invention with reference to the accompanying drawings, it is understood the invention is not limited to these precise embodiments, and various changes and modifications may be effected by one skilled in the art without departing from the scope of the invention as defined in the appended claims.

## Claims

1. A medical diode laser system having a single optical delivery fiber (18) wherein said delivery fiber has at least two core sections (15, 16) of different cross-sectional area to transmit radiation from at least two laser sources, wherein cross-sectional area of each core is selected independently, a smaller area for higher power density, to achieve separately a beam of laser light output (12) to cut tissue, and a larger area for lower power density, to achieve separately a laser light output (13) to coagulate or biostimulate tissue being cut.

2. A system according to claim 1, wherein said at least two laser sources are transmitted with at least two different power densities.

3. A system according to claim 1, wherein said at least two laser sources operate at at least two different wavelengths.

4. A system according to claim 1 wherein said radiation from a first source is pulsed and from a second source is a continuous wave.

5. A system according to claim 2 wherein said at least two laser sources also has at least two wavelengths.

6. A system according to claim 5, wherein said radiation from said at least two laser sources is transmitted simultaneously.

7. A system according to claim 1, wherein said fiber has at least two concentric cladding layers surrounding a single core and at least one of said cladding layers functions as a core.

8. A system according to claim 7, wherein an inner layer of said at least two concentric cladding layers is used to transmit radiation from a first of said at least two sources and the core transmits radiation from a second of at least two laser sources.

9. A system according to claim 1, wherein said fiber has at least two non-concentric cores within a cladding layer.

10. A system according to claim 9, wherein each of said at least two cores has a different cross-sectional area and each is used to transmit radiation from different laser sources.

11. A system according to claim 9, wherein said at least two cores' cross sectional shape is chosen from the group; circular, rectangular, or oval.

**12.** A system according to claim 1, wherein said fiber has a core surrounded by a cladding layer, which is further surrounded by an additional core layer, said additional core layer is surrounded by yet another cladding layer, and each layer has a progressively lower refractive index than that of said inner core.

**13.** A system according to claim 1 wherein said radiation from one of said at least two sources is used to coagulate tissue and radiation from the second of said at least two laser sources is used for ablating tissue.

**14.** A system according to claim 1, wherein said radiation from said at least two laser sources transmitted through said fiber, are more precisely directed to a treatment area using optics at a distal end of said medical laser system.

## Patentansprüche

**1.** Ein medizinisches Diodenlasersystem, **dadurch gekennzeichnet, dass** es eine einzige Faser **(18)** zur Übertragung optischer Strahlung hat, wobei besagte Faser mindestens zwei Faserkernbereiche **(15, 16)** mit unterschiedlichen Querschnittsflächen hat, um Strahlung von mindestens zwei Laserstrahlquellen zu übertragen, wobei die Querschnittsfläche jedes Kerns unabhängig gewählt ist; ein kleinerer Bereich für höhere Leistungsdichte, um separat einen Strahl mit einer Laserlichtleistung **(12)** zum Schneiden von Gewebe zu erreichen, und ein größerer Bereich für niedrigere Leistungsdichte, um separat eine Laserlichtleistung **(13)** zu erhalten, um das geschnittene Gewebe zu koagulieren oder zu biostimulieren.

**2.** Ein System nach Anspruch 1, wobei besagte mindestens zwei Laserstrahlquellen mit mindestens zwei unterschiedlichen Leistungsdichten übertragen werden.

**3.** Ein System nach Anspruch 1, wobei besagte mindestens zwei Laserstrahlquellen auf mindestens zwei unterschiedlichen Wellenlängen emittieren.

**4.** Ein System nach Anspruch 1, wobei besagte Strahlung von einer ersten Quelle gepulst und von einer zweiten Quelle kontinuierlich (engl.: continuous wave, CW) ist.

**5.** Ein System nach Anspruch 2, wobei besagte mindestens zwei Laserstrahlquellen auch mindestens zwei Wellenlängen haben.

**6.** Ein System nach Anspruch 5, wobei besagte Strahlung von besagten mindestens zwei Laserstrahlquellen simultan übertragen wird.

**7.** Ein System nach Anspruch 1, wobei besagte Faser mindestens zwei konzentrisch angeordnete Mantelschichten hat, die einen einzelnen Kern umgeben und wo mindestens eine der besagten Mantelschichten als Kern wirkt.

**8.** Ein System nach Anspruch 7, wobei eine innere Schicht besagter mindestens zwei konzentrischer Mantelschichten genutzt wird, um Strahlung von einer ersten der besagten mindestens zwei Quellen zu übertragen, und der Kern die Strahlung einer zweiten der mindestens zwei Laserstrahlquellen überträgt.

**9.** Ein System nach Anspruch 1, wobei besagte Faser mindestens zwei nicht-konzentrische Kerne innerhalb einer Mantelschicht besitzt.

**10.** Ein System nach Anspruch 9, wobei jeder der besagten mindestens zwei Kerne eine unterschiedliche Querschnittsfläche besitzt und jeder genutzt wird, um Strahlung von unterschiedlichen Laserstrahlquellen zu übertragen.

**11.** Ein System nach Anspruch 9, wobei die Form der Querschnitte besagter mindestens zwei Kerne aus der Gruppe zirkularer, rechteckiger und ovaler Formen ausgewählt wird.

**12.** Ein System nach Anspruch 1, wobei besagte Faser einen Kern hat, der von einer Mantelschicht umgeben ist, welche ihrerseits von einer zusätzlichen Kernschicht umgeben ist, und besagte zusätzliche Kernschicht von einer weiteren Mantelschicht umgeben ist, und jede Schicht einen stufenweise niedrigeren Brechungsindex als den des besagten inneren Kerns hat.

**13.** Ein System nach Anspruch 1, wobei besagte Strahlung von einer der besagten mindestens zwei Quellen genutzt wird, um Gewebe zu koagulieren und Strahlung der zweiten der besagten mindestens zwei Laserstrahlquellen genutzt wird, um Gewebe zu ablatieren.

14. Ein System nach Anspruch 1, wobei besagte Strahlung von besagten mindestens zwei Laserstrahlquellen, die durch besagte Faser übertragen wird, unter Verwendung von Optiken am distalen Ende des besagten medizinischen Lasersystems mit größerer Präzision auf das Behandlungsgebiet gerichtet wird.


**Revendications**

1. Un système médical de laser à diode qui dispose d'une seule fibre optique de transmission **(18)** où la fibre de transmission en question a au moins deux sections de coeur **(15, 16)** avec différentes aires de sections transversales pour transmettre la radiation d'au moins deux sources laser où l'aire de section transversale de chaque coeur est sélectionnée indépendamment, une aire plus petite pour une densité de puissance plus élevée, pour obtenir séparément un rayon de lumière laser **(12)** apte à sectionner les tissus, et une aire plus grande de moindre densité de puissance, pour obtenir séparément une puissance de sortie de la lumière laser **(13)** pour coaguler ou bio-stimuler les tissus venant d'être sectionnés.

2. Un système conformément à la revendication 1, où au moins deux sources laser en question sont transmises avec au moins deux densités de puissance différentes.

3. Un système conformément à la revendication 1, où au moins deux sources laser en question émettent avec au moins deux longueurs d'onde différentes.

4. Un système conformément à la revendication 1, où la radiation en question d'une première source est pulsée et celle d'une deuxième source est une onde continue (en anglais: continuous wave, CW).

5. Un système conformément à la revendication 2, où au moins deux sources laser en question ont également au moins deux longueurs d'onde.

6. Un système conformément à la revendication 5, où la radiation en question d'au moins deux sources laser en question est transmise simultanément.

7. Un système conformément à la revendication 1, où la fibre en question a au moins deux couches de gaine concentriques qui entourent un seul coeur et au moins une des couches de gaine en question fonctionne comme un coeur.

8. Un système conformément à la revendication 7, où une couche interne d'au moins deux couches de gaine concentriques en question est utilisée pour transmettre la radiation d'une première d'au moins deux sources et le coeur transmet la radiation d'une seconde d'au moins deux sources laser.

9. Un système conformément à la revendication 1, où la fibre en question a au moins deux coeurs non concentriques dans une couche de gaine.

10. Un système conformément à la revendication 9, où chacun des au moins deux coeurs en question a une aire de section transversale différente et chacun est utilisé pour transmettre la radiation de sources laser différentes.

11. Un système conformément à la revendication 9, où la forme de la section transversale d'au moins deux coeurs en question est sélectionné du groupe; circulaire, rectangulaire et ovale.

12. Un système conformément à la revendication 1, où la fibre en question a un coeur entouré par une couche de gaine qui est encore entourée par une couche de coeur supplémentaire, laquelle couche de coeur est entourée par une couche de gaine supplémentaire, et chaque couche a un indice de réfraction qui est progressivement inférieure à celle du coeur interne en question.

13. Un système conformément à la revendication 1, où la radiation en question de l'une des au moins deux sources en question est utilisée pour coaguler les tissus et la radiation de la seconde des au moins deux sources laser en question est utilisée pour ablater les tissus.

**14.** Un système conformément à la revendication 1, où la radiation en question des au moins deux sources laser en question, transmis par la fibre en question, est plus précisément dirigée vers la région de traitement, en utilisant des optiques au bout périphérique du système laser médical en question.

FIGURE 1

FIGURE 2

FIGURE 3

FIGURE 4

FIGURE 5

FIGURE 6

FIGURE 7

FIGURE 8

FIGURE 9

EP 1 284 672 B1

FIGURE 10

EP 1 284 672 B1